(19) Europäisches Patentamt
European Patent Office
Office européen des brevets

(11) Veröffentlichungsnummer: **0 256 344 B1**

(12) **EUROPÄISCHE PATENTSCHRIFT**

(45) Veröffentlichungstag der Patentschrift: **22.04.92**

(51) Int. Cl.$^5$: **A61K 39/44**, G02C 7/04

(21) Anmeldenummer: **87110671.2**

(22) Anmeldetag: **23.07.87**

# (54) Kontaktlinse.

(30) Priorität: **18.08.86 DE 3627937**

(43) Veröffentlichungstag der Anmeldung:
**24.02.88 Patentblatt 88/08**

(45) Bekanntmachung des Hinweises auf die Patenterteilung:
**22.04.92 Patentblatt 92/17**

(84) Benannte Vertragsstaaten:
**CH DE FR GB IT LI**

(56) Entgegenhaltungen:
**EP-A- 0 088 606**
**WO-A-86/07264**

(73) Patentinhaber: **Müller-Lierheim, Wolfgang, Dr.,**
**Lichtweg 5**
**W-8032 Gräfelfing(DE)**

(72) Erfinder: **Müller-Lierheim, Wolfgang, Dr.,**
**Lichtweg 5**
**W-8032 Gräfelfing(DE)**

(74) Vertreter: **Nöth, Heinz, Dipl.-Phys.**
**Patentanwälte Pfenning, Meinig & Partner**
**Mozartstrasse 17**
**W-8000 München 2(DE)**

**EP 0 256 344 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Kontaktlinsen werden üblicherweise aus inerten, untoxischen, optisch klaren und schlierenfreien Kunststoffen hergestellt. Durch Auswahl geeigneter Polymere oder nachträgliche Oberflächenbehandlung werden sie für wäßrige Lösungen benetzbar gemacht. Um den Stoffwechsel der unter der Kontaktlinse liegenden Hornhaut möglichst wenig zu beeinträchtigen, kommen heutzutage vorzugsweise dünne Kontaktlinsen aus gasdurchlässigen Polymeren zur Anwendung.

Probleme des Kontaktlinsentragens betreffen vor allem

- den mechanischen Reiz an Lid und Hornhaut,
- die Verschmutzung, Reinigung und Desinfektion der Kontaktlinse und
- die durch Kontaktlinsentragen verursachte Verringerung der Tränenfilmstabilität.

An den mechanischen Reiz kann sich der Kontaktlinsenträger je nach Kontaktlinsentyp innerhalb weniger Stunden bis längstens Wochen gewöhnen.

Der Verschmutzung der Kontaktlinse durch unspezifische Adsorption von Lipiden, Proteinen, Mucinen und Salzen des Tränenfilms sowie anderer Moleküle und Partikel, einschließlich Mikroorganismen, wird üblicherweise durch regelmäßige Reinigung und Desinfektion begegnet. Zur Reinigung der Kontaktlinse kommen neben Detergentien auch proteolytische Enzyme zur Anwendung. Die von der Kontaktlinse nicht vollständig entfernten Proteine werden durch Reinigung und chemische oder Hitzedesinfekttion häufig denaturiert und führen zu allergischen Reaktionen der Konjunktiva des Kontaktlinsenträgers.

Der Tränenfilm des menschlichen Auges ist, vereinfacht dargestellt, in drei Schichten aufgebaut,

- einer Mucinschicht, die die rauhe Oberfläche des Hornhautepithels glättet und das hydrophobe Hornhautepithel benetzbar macht,
- einer wäßrigen Phase und
- einer Lipidschicht, die die wäßrige Phase gegen die umgebende Luft isoliert.

Die Lipidschicht des Tränenfilms verhindert ein rasches Verdunsten der wäßrigen Phase und eine zu rasche Abkühlung der Hornhaut.

Das Tragen herkömmlicher Kontaktlinsen bedingt eine Verringerung der Tränenfilmstabilität und als Folge davon ein rascheres Verdunsten der wäßrigen Phase des Tränenfilms. Kontaktlinsenträger leiden daher häufig unter trockenen Augen und müssen mitunter Benetzungslösungen ins Auge einträufeln. Keineswegs selten führt die Tränenfilminstabilität zu einer Unverträglichkeit der Kontaktlinse.

Aufgabe der Erfindung ist es daher, eine Kontaktlinse zu schaffen, bei der die Augenverträglichkeit der Oberfläche dahingehend verbessert ist, daß sie den Eigenschaften der Konjunktiva möglichst nahe angepaßt ist.

Diese Aufgabe wird erfindungsgemäß dadurch gelöst, daß an der Linsenoberfläche monoklonale Antikörper oder Antikörperfragmente immobilisiert sind, deren N-terminale Enden gegen-Mucussubstanzen des menschlichen Augentränenfilms gerichtet sind.

Hierdurch wird erreicht, daß an der Kontaktlinsenoberfläche Moleküle nicht unspezifisch adsorbiert werden, sondern bestimmte Epitope ausgewählter Moleküle gebunden werden, die auch an der Oberfläche der Konjunktiva des menschlichen Auges vorhanden sind. Bei diesen Substanzen, welche von den monoklonalen Antikörpern an der Linsenoberfläche bevorzugt gebunden werden, handelt es sich bevorzugt um solche Mucussubstanzen, die vom Epithel, insbesondere den Gobletzellen, der Konjunktiva des menschlichen Auges abgesondert werden.

Hierbei werden Antikörper bzw. Antikörperfragmente bevorzugt, deren N-terminale Enden zu den Mucussubstanzen bei einem pH-Wert der Tränenflüssigkeit, d. h. bei ca. 7,0 - 7,5, ein Bindungsmaximum besitzen und die Affinität zu den gebundenen Molekülen bei einer pH-Änderung so weit herabgesetzt wird, daß sie desorbieren, so daß eine schonende Reinigung der Kontaktlinse erreicht wird.

Hierdurch wird erreicht, daß auch auf der Kontaktlinsenoberfläche eine Schleimschicht (Mucus) erzeugt wird, welche die gewünschte Benetzbarkeit mit dem Tränenfilm herstellt. Dadurch, daß man im Bereich der N-terminalen Enden die Antikörper bzw. Antikörperfragmente hydrophob gestaltet, besitzt die Oberfläche des Kontaktlinsenkörpers die gleiche Eigenschaft wie das Hornhautepithel, wobei jedoch durch die Schleimschicht, welche von den Antikörpern an der Linsenkörperoberfläche gebunden ist, die gute Benetzbarkeit mit der Tränenflüssigkeit vermittelt wird.

Um die Gefahr eines Aufreißens des Tränenfilms zu vermeiden, kreuzreagieren die monoklonalen Antikörper mit den in der Tränenflüssigkeit vorkommenden Lipiden nicht.

Bevorzugt werden Antikörper bzw. Antikörperfragmente an die Oberfläche des Kontaktlinsenkörpers gebunden, die an ihren N-terminalen Enden Plasmaproteine des menschlichen Augentränenfilms adsorbieren. Diese Proteine, die unter anderen Albumin und Alpha-, Beta-, Gammaglobulin sowie Lysozym enthalten (Chien-chyou W. Chao and Stuart I. Brown "Macromolecular Components of Human Ocular Mucus" in "The

preocular Tear Film", 1986, Dry Eye Institute, Lubbock, Texas, Seiten 331 bis 340), bilden einen Hauptbestandteil der Mucusschicht.

Die Plasmaproteine, insbesondere Albumin, dienen als Gleitsubstanzen und als Benetzungsvermittler in der Tränenflüssigkeit. Die Schleimschicht (Mucus) auf dem menschlichen Auge besteht im wesentlichen aus Mucosubstanzen (ca. 40 %) mit einem Molekulargewicht von mehr als $10^5$ Dalton und den Plasmaproteinen (ca. 60 %) mit einem Molekulargewicht von weniger als $10^5$ Dalton. Die Mucosubstanzen sind im wesentlichen komplexe Glycokonjugate, die aus Glycoproteine und Proteoglycane bestehen (Tabelle 1, Seite 333 der obengenannten Veröffentlichung).

Zur Gewinnung der Antikörper eignen sich herkömmliche Hybridoma-Techniken. Hierzu werden die monoklonalen Antikörper aus Hybridomazellen erhalten, die Fusionsprodukte von Mausmilzzellen mit Myelomzellen sind. Die Mausmilzen stammen von Tieren, bevorzugt Balb/c-Mäusen, die mit den entsprechenden Mucussubstanzen immunisiert worden sind.

Die Mäuse werden über einen Zeitraum von vier bis sechs Monaten einmal monatlich mit den gegebenen Mucussubstanzen immunisiert. Zeigt eine Maus einen hinreichenden Anikörpertiter, der mittels der ELISA (enzyme linked immunosorbent assay)-Technik festgestellt wird, erfolgt eine Boosterinjektion mit den gegebenen Mucussubstanzen. Die Maus wird getötet und die steril entnommenen Milzzellen mit Myelomzellen (z. B. Zell-Linie P3X63-Ag8.653,ATCC CRL 1580) fusioniert.

Die entstehenden Hybridisierungsprodukte werden in HAT-Standard-Selektionsmedium (Hypoxanthin, Aminopterin, Thymidin) selektiert.

Primärkulturen, die im Überstand eine positive Reaktion auf die Mucussubstanzen zeigen, werden kloniert. Die monoklonalen Antikörper dieser Klone werden, insbesondere im Hinblick auf Kreuzreaktionen mit Lipiden im Tränenfilm und Keimen der Konjunktiva, auf Spezifität getestet. Die ausgewählten monoklonalen Antikörper, die für die verschiedenen Mucussubstanzen spezifisch sind, werden beispielsweise durch Aszitesproduktion in den erforderlichen Mengen hergestellt.

Hierzu erhalten Balb/c-Mäuse in wöchentlichem Abstand 2 x 0,5 ml Pristan intraperitoneal. Pro Maus werden 5 x $10^6$ Hybridomzellen der ausgewählten Klone appliziert. Die Aszitesgewinnung erfolgt nach ca. 11 Tagen.

Von den Antikörpern bzw. Antikörperfragmenten, die gegen Plasmaproteine gerichtet sind, sind bevorzugt ca.
25 bis 30 % gegen Albumin
25 bis 30 % gegen Beta-Globulin
20 bis 30 % gegen Lysozym
3 bis 10 % gegen Alpha-Globulin
3 bis 6 % gegen Gamma-Globulin
gerichtet.

Die gewonnenen Antikörper werden an der Oberfläche von herkömmlichen Kontaktlinsenkörpern kovalent gebunden. Die Oberflächen der Kontaktlinsenkörper besitzen bindungsaktive chemische Gruppen, die insbesondere durch Copolymerisation von geeigneten Monomeren oder Oligomeren gebildet sind. Es kann sich hierbei um -SH, -OH, $NH_2$, $-C\equiv N$ oder um

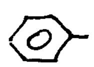

Gruppen handeln. Bevorzugt werden Oxirangruppen zur Immobilisierung der Antikörper an der Kontaktlinsenoberfläche verwendet. Beispiele für die Immobilisierung der Antikörper an den Kontaktlinsenoberflächen sind folgende:

**Beispiel 1**

Aminogruppen tragende Trägeroberflächen lassen sich z. B. mit Thiophosgen aktivieren. Als aktivierende Spezies entsteht dabei ein Isothiocyanat am Träger.

Trägeraktivierung

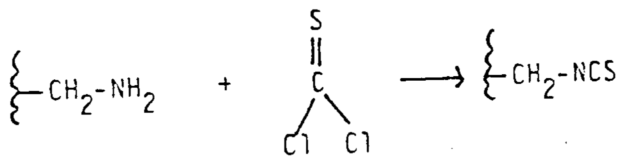

Träger Thiophosgen aktivierter Träger

Antikörper-Immobiliserung

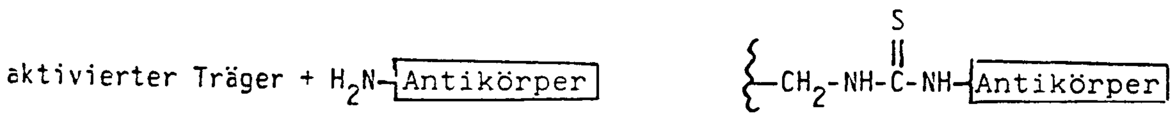


immobilisierter Antikörper

**Beispiel 2**

Ein breites Reaktionsspektrum bietet die Epichlorhydrinmethode. Bei ihr wird die Trägeroberfläche mit Hilfe von Epichlorhydrin mit oxirangruppenhaltigen Spacern gespickt. Die unter Ringspannung stehenden Epoxygruppen lassen sich unter sehr milden Reaktionsbedingungen nukleophil mit Thiol-, Hydroxyl- oder Aminogruppen öffnen.

Trägeraktivierung

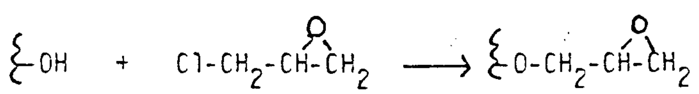

Träger Epichlorhydrin aktivierter Träger

Antikörper-Immobilisierung

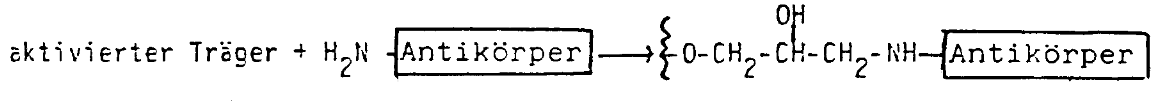


immobilisierter Antikörper

**Beispiel 3**

Die Effektivität einer Immobilisierung hängt nicht nur von der Wahl der Verknüpfungsmethode, sondern auch vom Abstand Träger zu Protein ab.

Solchen Gesichtspunkten trägt z.B. die Bisepoxioxiran-Methode Rechnung. Hierbei wird eine Oxirangruppe zur Verankerung am Träger und die zweite zur Fixierung des Proteins verwendet.

Trägeraktivierung

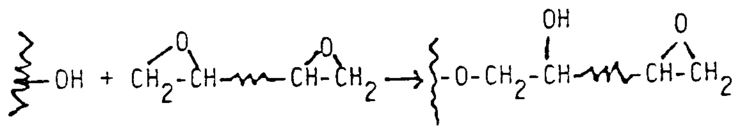

Träger Bisepoxioxiran aktivierter Träger

Antikörper-Immobilisierung

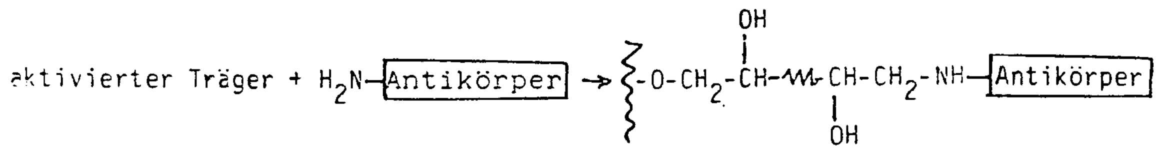

immobilisierter Antikörper

Bei dem Kontaktlinsenmaterial kann es sich um hartes Kontaktlinsenmaterial, beispielsweise Polymethylmethacrylat, oder auch um weiches Kontaktlinsenmaterial, beispielsweise Polyhydroxyethylmethacrylat, oder Polyethylenglycoldimethacrylat handeln.

Die an der Kontaktlinsenoberfläche immobilisierten monoklonalen Antikörper weisen keine Kreuzreaktionen zu Keimen auf, welche die Bindehaut und die tränenabführenden Wege besiedeln. Es handelt sich hier im wesentlichen um die folgenden Mikroorganismen:

Staphylococcus aureus Aspergillus niger
Corynebacterium xerosis Candida albicans
Streptokokken Penicillium
Bacillus subtilis
Pseudomonas aeruginosa
Neisseria
Sarcina
Enterobakterien wie
E. coli
E. freundii
Klebsiella
Proteus spec.

**Patentansprüche**

**1.** Kontaktlinse mit einer augenverträglichen Oberfläche, dadurch gekennzeichnet, daß an der Linsenoberfläche monoklonale Antikörper oder Antikörperfragmente immobilisiert sind, deren N-terminale Enden gegen Mucussubstanzen des menschlichen Augentränenfilms gerichtet sind.

**2.** Kontaktlinse nach Anspruch 1, dadurch gekennzeichnet, daß die Antikörper bzw. Antikörperfragmente gegen Mucussubstanzen, die von Epithel der Konjunktive abgesondert werden, gerichtet sind.

**3.** Kontaktlinse nach Anspruch 1 oder 2, dadurch gekennzeichnet, daß die Antikörper bzw. Antikörperfragmente bei einem pH-Wert (ca. 7,0 - 7,5) der Tränenflüssigkeit ein Bindungsmaximum besitzen.

**4.** Kontaktlinse nach einem der Ansprüche 1 bis 3, dadurch gekennzeichnet, daß der Bereich der N-terminalen Enden der Antikörper bzw. der Antikörperfragmente hydrophob ist.

**5.** Kontaktlinse nach einem der Ansprüche 1 bis 4, dadurch gekennzeichnet, daß die Antikörper bzw.

Antikörperfragmente mit Lipiden des menschlichen Augentränenfilms nicht kreuzreagieren.

**6.** Kontaktlinse nach einem der Ansprüche 1 bis 5, dadurch gekennzeichnet, daß die Antikörper bzw. Antikörperfragmente gegen Plasmaproteine des menschlichen Augentränenfilms gerichtet sind.

**7.** Kontaktlinse nach Anspruch 6, dadurch gekennzeichnet, daß die Antikörper bzw. Antikörperfragmente gegen Albumin gerichtet sind.

**8.** Kontaktlinse nach Anspruch 6 oder 7, dadurch gekennzeichnet, daß von den Antikörpern bzw. Antikörperfragmenten, die gegen Plasmaproteine gerichtet sind, ca.
25 bis 30 % gegen Albumin
25 bis 30 % gegen Beta-Globulin
20 bis 30 % gegen Lysozym
3 bis 10 % gegen Alpha-Globulin
3 bis 6 % gegen Gamma-Globulin
gerichtet sind.

**9.** Kontaktlinse nach einem der Ansprüche 1 bis 5, dadurch gekennzeichnet, daß die Antikörper bzw. Antikörperfragmente gegen Glykokonjugate des menschlichen Augentränenfilms gerichtet sind.

**10.** Kontaktlinse nach einem der Ansprüche 1 bis 9, dadurch gekennzeichnet, daß die Antikörper kovalent an die Kontaktlinsenoberfläche gebunden sind.

**11.** Kontaktlinse nach einem der Ansprüche 1 bis 10, dadurch gekennzeichnet, daß die Oberfläche des Linsenkörpers über -SH, -OH, $-NH_2$, -C≡N oder

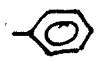

Gruppen aktiviert ist und die Antikörper bzw. Antikörperfragmente über diese Gruppen kovalent an die Linsenoberfläche gebunden sind.

**12.** Kontaktlinse nach einem der Ansprüche 1 bis 11, dadurch gekennzeichnet, daß die aktiven chemischen Gruppen an der Oberfläche des Linsenkörpers durch Copolymerisation von geeigneten Monomeren oder Oligomeren gebildet sind.

**13.** Kontaktlinse nach einem der Ansprüche 1 bis 12, dadurch gekennzeichnet, daß die Antikörper bzw. Antikörperfragmente mit auf der menschlichen Konjunktiva oder in den tränenführenden Wegen vorkommenden Keimen nicht kreuzreagieren.

**Claims**

**1.** A contact lens with an eye-compatible surface characterised in that monoclonal antibodies or antibody fragments are immobilised at the lens surface, the N-terminal ends of the antibodies or antibody fragments being directed against mucous substances of the human eye tear film.

**2.** A contact lens according to claim 1 characterised in that the antibodies or antibody fragments are directed against mucous substances which are secreted by the epithelium of the conjunctiva.

**3.** A contact lens according to claim 1 or claim 2 characterised in that the antibodies or antibody fragments have a binding maximum at a pH-value (about 7.0 - 7.5) of the lachrymal fluid.

**4.** A contact lens according to one of claims 1 to 3 characterised in that the area of the N-terminal ends of the antibodies or antibody fragments is hydrophobic.

**5.** A contact lens according to one of claims 1 to 4 characterised in that the antibodies or antibody fragments do not cross-react with lipids of the human eye tear film.

6. A contact lens according to one of claims 1 to 5 characterised in that the antibodies or antibody fragments are directed against plasma proteins of the human eye tear film.

7. A contact lens according to claim 6 characterised in that the antibodies or antibody fragments are directed against albumin.

8. A contact lens according to claim 6 or claim 7 characterised in that of the antibodies or antibody fragments which are directed against plasma proteins,
about 25 to 30% are directed against albumin
about 25 to 30% are directed against beta globulin
about 20 to 30% are directed against lysozyme
about 3 to 10% are directed against alpha globulin
about 3 to 6% are directed against gamma globulin.

9. A contact lens according to one of claims 1 to 5 characterised in that the antibodies or antibody fragments are directed against glycoconjugates of the human eye tear film.

10. A contact lens according to one of claims 1 to 9 characterised in that the antibodies are covalently bound to the surface of the contact lens.

11. A contact lens according to one of claims 1 to 10 characterised in that the surface of the lens body is activated by way of -SH, -OH, -$NH_2$, -C≡N or

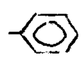

and the antibodies or antibody fragments are covalently bound to the lens surface by way of said groups.

12. A contact lens according to one of claims 1 to 11 characterised in that the active chemical groups on the surface of the lens body are formed by copolymerisation of suitable monomers or oligomers.

13. A contact lens according to one of claims 1 to 12 characterised in that the antibodies or antibody fragments do not cross-react with germs occurring on the human conjunctiva or in the tear ducts.

**Revendications**

1. Verre de contact avec une surface tolérée par l'oeil, caractérisé en ce que sur la surface du verre sont immobilisés des anticorps ou fragments d'anticorps monoclonaux dont les fonctions terminales N sont dirigées sur des substances muqueuses de la pellicule lacrymale de l'oeil humain.

2. Verre de contact selon la revendication 1, caractérisé en ce que les anticorps ou fragments d'anticorps sont dirigés sur des substances muqueuses qui se séparent de l'épithélium de la conjonctive.

3. Verre de contact selon la revendication 1 ou 2, caractérisé en ce que les anticorps ou fragments d'anticorps présentent un maximum de liaison pour un pH d'environ 7,0 à 7,5 du liquide lacrymal.

4. Verre de contact selon l'une quelconque des revendications précédentes, caractérisé en ce que la région des fonctions terminales N des anticorps ou des fragments d'anticorps est hydrophobe.

5. Verre de contact selon l'une quelconque des revendications précédentes, caractérisé en ce que les anticorps ou fragments d'anticorps n'entrent pas en réaction croisée avec des lipides de la pellicule lacrymale de l'oeil humain.

6. Verre de contact selon l'une quelconque des revendications précédentes, caractérisé en ce que les anticorps ou fragments d'anticorps sont dirigés sur des protéines plasmatiques de la pellicule lacrymale de l'oeil humain.

**7.** Verre de contact selon la revendication 6, caractérisé en ce que les anticorps ou fragments d'anticorps sont dirigés sur de l'albumine.

**8.** Verre de contact selon la revendication 6 ou 7, caractérisé en ce que parmi les anticorps ou fragments d'anticorps dirigés sur des protéines plasmatiques sont dirigés d'entre eux environ:
25 à 30 % sur l'albumine
25 à 30 % sur la bêta-globuline
20 à 30 % sur la lysozyme
3 à 10 % sur l'alpha-globuline
3 à 6 % sur la gamma-globuline.

**9.** Verre de contact selon l'une quelconque des revendications 1 à 5, caractérisé en ce que les anticorps ou fragments d'anticorps sont dirigés sur des glycoconjugués de la pellicule lacrymale de l'oeil humain.

**10.** Verre de contact selon l'une quelconque des revendications précédentes, caractérisé en ce que les anticorps sont liés de façon covalente à la surface du verre de contact.

**11.** Verre de contact selon l'une quelconque des revendications précédentes, caractérisé en ce que la surface du corps du verre est activée par l'intermédiaire de groupes -SH, -OH, -$NH_2$, -C≡N ou

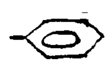

et en ce que les anticorps ou fragments d'anticorps sont liés par l'intermédiaire de ces groupes de façon covalente à la surface du verre.

**12.** Verre de contact selon l'une quelconque des revendications précédentes, caractérisé en ce que les groupes chimiques actifs à la surface du corps de verre sont formés par copolymérisation de mononères ou oligomères appropriés.

**13.** Verre de contact selon l'une quelconque des revendications précédentes, caractérisé en ce que les anticorps ou fragments d'anticorps n'entrent pas en réaction croisée avec des germes présents sur la conjonctive humaine ou dans les conduits lacrymaux.